(19) Europäisches Patentamt

European Patent Office

Office européen des brevets

(11) **EP 0 484 369 B1**

(12) **EUROPEAN PATENT SPECIFICATION**

(45) Date of publication and mention
of the grant of the patent:
**14.11.2001 Bulletin 2001/46**

(51) Int Cl.[7]: **C07K 14/00**, C12N 15/12,
C12Q 1/66, C12Q 1/68,
G01N 33/542

(21) Application number: **90910819.3**

(22) Date of filing: **23.07.1990**

(86) International application number:
**PCT/GB90/01131**

(87) International publication number:
**WO 91/01305 (07.02.1991 Gazette 1991/04)**

(54) **MODIFIED BIOLUMINESCENT PROTEINS AND THEIR USE**

MODIFIZIERTE BIOLUMINESZIERENDE PROTEINE UND DEREN VERWENDUNG

PROTEINES BIOLUMINESCENTES MODIFIEES ET LEUR UTILISATION

(84) Designated Contracting States:
**AT BE CH DE DK ES FR GB IT LI LU NL SE**

(30) Priority: **22.07.1989 GB 8916806**

(43) Date of publication of application:
**13.05.1992 Bulletin 1992/20**

(60) Divisional application:
**00203862.8 / 1 097 992**

(73) Proprietor: **University of Wales College of
Medicine
Cardiff CF4 4XN (GB)**

(72) Inventor: **CAMPBELL, Anthony, Keith
14 Maillard's Haven
S. Glamorgan CF6 2RF (GB)**

(74) Representative: **Newell, William Joseph et al
Wynne-Jones, Lainé & James
Morgan Arcade Chambers
33 St. Mary Street
Cardiff, CF10 1AF (GB)**

(56) References cited:
**EP-A- 0 103 469          EP-A- 0 137 515**

- **Proc. Natl. Acad. Sci. USA, Volume 83, No. 21,
November 1986 F.I. TSUJI et al.: "Site-Specific
Mutagenesis of the Calcium-Binding
Photoprotein Aequorin", pages 8107-8111 see
the whole article**

- **Proc. Natl. Acad. Sci. USA, Volume 86, No. 1,
January 1989 K. KUROSE et al.:
"Bioluminescence of the Ca2+-Binding
Photoprotein Aequorin after Cysteine
Modification" see the whole article**
- **CHEMICAL ABSTRACTS, Volume 104, No. 17, 28
April 1986, (Columbus, Ohio, US), S.C. ALTER et
al.: "The Sulfhydryls of Firefly Luciferase are not
Essential for Activity" see pages 301-302,
Abstract No. 144584g & Biochemistry 1986, 25
(7) 1599-605**
- **CHEMICAL ABSTRACTS, Volume 83, 1975,
(Columbus, Ohio, US), T.W. CLINE et al.:
"Mutated Luciferases with Altered
Bioluminescence Emission Spectra" see page
253, Abstract No. 160401y & J. Biol. Chem. 1974,
249 (14), 4668-9**
- **CHEMICAL ABSTRACTS, Volume 76, No. 1, 3
January 1972, (Columbus, Ohio, US), E.A.
MEIGHEN et al.: "Hybridization of Bacterial
Luciferase with a Variant Procuded by Chemical
Modification" see page 131, Abstract No. 1279d
& Bio Chemistry 1971, 101 (22), 4062-8**

Note: Within nine months from the publication of the mention of the grant of the European patent, any person may give
notice to the European Patent Office of opposition to the European patent granted. Notice of opposition shall be filed in
a written reasoned statement. It shall not be deemed to have been filed until the opposition fee has been paid. (Art.
99(1) European Patent Convention).

- CHEMICAL ABSTRACTS, Volume 112, No. 23, 4 June 1990, (Columbus, Ohio, US), W.H.R. LANGRIDGE et al.: "Use of Low Light Image Microscopy to Monitor Genetically Engineered Bacterial Luciferase Gene Expression in Living Cells and Gene Activation Throughout the Development of a Transgenic Organism" see pages 295-296, Abstract No. 213228y & Proc. SPIE-Int. Soc. Opt. 1089, 1161 (New Methods Microsc. Low Light Imaging), 216-29

- CHEMICAL ABSTRACTS, Vol. 113, No. 3, 16 July 1990, (Columbus, Ohio, US), T.M. JENKINS et al.: "Measurement of Protein Phosphorylation by Covalent Modification of Firefly Luciferase" see page 315, Abstract No. 20373d & Biochem. Soc. Trans. 1990, 18 (3), 463-4

**Description**

This invention relates to bioluminescent proteins, in particular it relates to bioluminescent proteins which have been modified, for example by chemical means or by genetic engineering. Such modified bioluminescent proteins, hereinafter referred to as "rainbow proteins", may be used in the detection and quantification of cells, microbes such as bacteria, viruses and protozoa, and substances of biological interest such as substrates, metabolites, intra- and extra- cellular signals, enzymes, antigens, antibodies and nucleic acids.

Bioluminescence is the oxidation of an organic molecule, the "luciferin", by oxygen or one of its netabolites, to emit light. The reaction is catalysed by a protein, usually known as a "luciferase", or a "photoprotein" when the luciferin is so tightly or covalently bound to the luciferase that it does not diffuse off into the surrounding fluid.

$$O_2 + luciferin + luciferase \rightarrow oxyluciferin + light$$

$$(or\ O_2^- \ or\ H_2O_2)\ (or\ photoprotein)$$

Up to three other substances may also be required to be present in order to generate light, or to alter its colour, and they are as follows:-

(a) A cation such as $H^+$, $Ca^{2+}$, $Mg^{2+}$, or a transition metal such as $Cu^+/Cu^{2+}$, $Fe^{2+}/Fe^{3+}$.
(b) A cofactor such as NADH, FMN, or ATP.
(c) A fluor as an energy transfer acceptor.

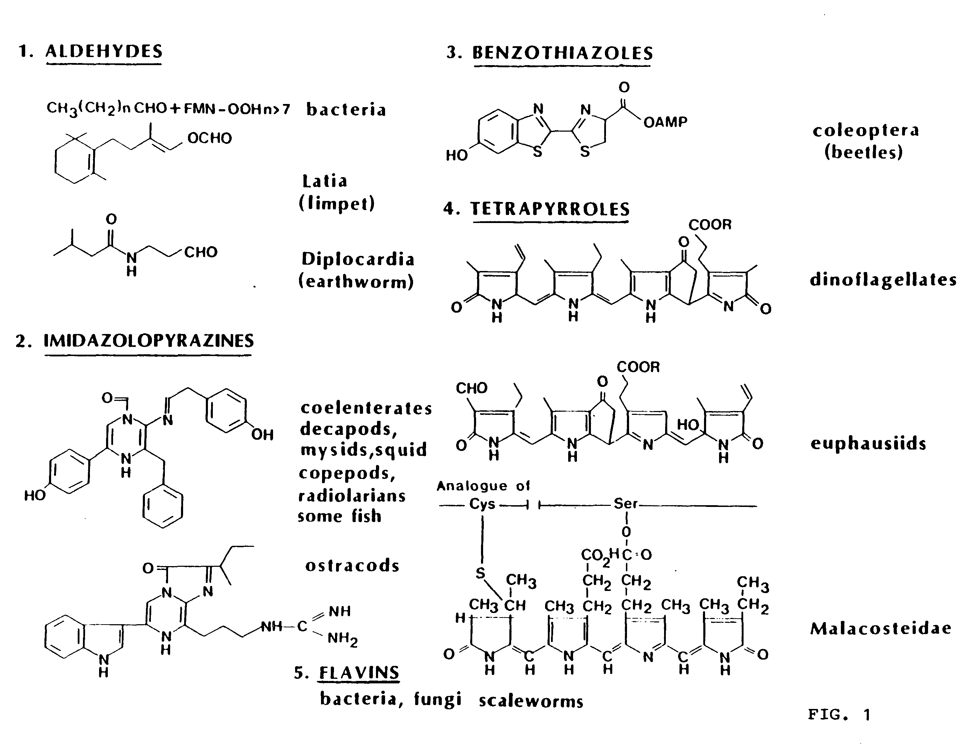

Five chemical families of luciferin have been identified so far (see Figure 1 of the attached drawing):

(a) Aldehydes (found in bacteria, freshwater limpet Latia and earthworms).
(b) Imidazolopyrazines (found in Sarcomastigophora, Cnidaria, Ctenophora, some Arthropoda, some Mollusca, some Chordata).
(c) Benzothiazole (found in beetles such as fireflies and glowworms).
(d) Linear tetrapyrroles (found in dinoflagellates, euphasiid shrimp, some fish).
(e) Flavins (found in bacteria, fungi, polychaete worms and some molluscs).

Reactions involving these luciferins may result in the emission of violet, blue, blue-green, green, yellow or red light and occasionally UV or IR light and such emission may or may not be linearly or circularly polarised. Reference is directed to Chemiluminescence principles and applications in biology and medicine, A.K. Campbell, publ. 1988 Horwood/VCH Chichester Weinheim, for further discussion of bioluminescent reactions.

Tsuji, F.I. et al (PNAS USA Vol.83, pp 8107-8111,1986) describe a modified aequorin in which amino acid substitutions were made at each of the three $Ca^{2+}$ binding sites, the three cysteines and a histidine in one of the hydrophobic regions. They show that the aequorin lost all of its activity when the substitution of arginine for glycine was made at the $Ca^{2+}$ binding site at position 29, it lost approximately half its activity when the substitution was made at position 122, and there was no loss of activity when the same substitution was made at position 158.

EP-A-103469 discloses an arrangement in which the reaction kinetics of an immunosassay in which a chemiluminescent label is linked to an immunoreactant such as an antigen, an antibody, a hapten etc are monitored. The chemiluminescent reaction is triggered and the rate of the reaction monitored to determine the amount of analyte.

EP-A-137515 discloses a homogenous energy transfer assay in which one ligand is covalently linked to a photoprotein and the other ligand is covalently linked to an energy transfer acceptor.

It has now been found that the light emitted from a bioluminescent reaction involving a modified bioluminescent or "rainbow" protein, may be changed in intensity, colour or polarisation. Such a change can then be used in various assays for detecting, locating and measuring cells, microbes and biological molecules.

In embodiments of this invention, the cell or substance causes a physical or chemical change, such as phosphorylation, to a rainbow protein as characterised in claims 1, 2 or 4 such as a genetically engineered luciferase, resulting in a change in intensity, colour or polarisation of the light emission. The bioluminescent reaction is triggered by adding, for example, the luciferin, and modification of the luciferase by the cell or substance being measured causes the reaction to emit light at a shorter or longer wavelength. This enables specific reactions to be detected and quantified in live cells, and within organelles or on the inner or outer surface of the plasma membrane, without the need to break them open, and without the need for separation of bound and unbound fractions.

Accordingly, in one aspect, this invention provides a bioluminescent protein selected from the group comprising firefly luciferase, aequorin or obelin, modified to include at the N or C terminus or within the protein a recognition site for a selected analyte, said recognition site being selected from the group comprising kemptide, malantide and an RRXS phosphorylation site, and not being present in the unmodified protein, wherein the analyte is capable of interacting with said recognition site, the modified bioluminescent protein being reactive in a bioluminescent reaction to produce light or other radiation, wherein the light or other radiation is produced having a physical property of a first characteristic when the analyte is interacting with the recognition site, and is produced having a second different characteristic of said physical property when the analyte is not interacting with said recognition site.

In another aspect, this invention provides a bioluminescent protein selected from the group comprising firefly luciferase, aequorin and obelin, modified to include at the N or C terminus or within the protein a recognition site responsive to the ambient conditions, said recognition site being selected from the group comprising kemptide, malantide and an RRXS phosphorylation site, and not being present in the unmodified protein, the modified bioluminescent protein being reactive in a bioluminescent reaction to produce light or other radiation, wherein the light or other radiation produced has a physical property of a first characteristic when the recognition site is exposed to a first condition, and has a second different characteristic when said recognition site is exposed to a second condition.

Preferably said first and second characteristics include at least one of colour, polarization state and intensity.

In a further aspect, this invention provides a bioluminescent protein selected from the group comprising firefly luciferase, aequorin and obelin, modified to include at the N or C terminus or within the protein a recognition site responsive to the concentration of a substance of interest, said site being selected from the group comprising kemptide, malantide and an RRXS phosphorylation site, and not being present in the unmodified protein, said modified bioluminescent protein being reactive in a bioluminescent reaction wherein light or other radiation emitted in a bioluminescent reaction involving said protein is produced in a varying physical characteristic depending on the concentration of said substance of interest.

Said bioluminescent protein may include a signal peptide for targeting the protein to a preselected organelle or site within the cell.

In one example, the bioluminescent protein comprises firefly luciferase modified to include kemptide or malantide covalently bound to the N or C terminus thereof.

In another example, the bioluminescent protein may comprise firefly luciferase modified to include an RRXS phophorylation site covalently intermediate the N and C terminus.

Yet further, the bioluminescent protein may comprise aequorin modified to include kemptide or malantide covalently bound at the N terminus.

In another aspect, this invention provides a method of detecting, locating or measuring a substance of interest, the method comprising:-

(a) providing a bioluminescent protein as described herein in a reaction system containing an analyte of interest,
(b) causing said bioluminescent protein to take part in a bioluminescent reaction, and
(c) analyzing the characteristics of light or other radiation produced, and
(d) detecting, locating or measuring said analyte, based on said characteristics.

The invention also provides a method of detecting a change in a physical, chemical, biochemical or biological condition, the method comprising:-

(a) providing a bioluminescent protein as described herein in a reaction system of interest;
(b) causing said bioluminescent protein to take part in a bioluminescent reaction;
(c) observing whether light or other radiation of the first and/or second characteristic is produced, and
(d) detecting said change based on production of light or radiation of said first or second characteristic.

The invention also provides a method of detecting, locating or measuring a substance of interest within a cell, or monitoring at least one of the physical, chemical, biochemical or biological conditions within a cell, which method comprises:

(a) introducing into said cell nucleic acid coding for a bioluminescent protein as described herein,
(b) causing said bioluminescent protein to be expressed;
(c) causing said modified bioluminescent protein to take part in a bioluminescent reaction, and
(d) analyzing the characteristics of the light or other radiation produced.

The invention also extends to nucleic acid coding for the bioluminescent protein as described above and to such nucleic acids including a promoter or enhancer sequence specific to a tissue or substance of interest.

The rainbow protein may be produced by the alteration, substitution, addition or removal of one or more amino acids from the end of or within the luciferase or photoprotein. As a result the light emission from the oxyluciferin may be of different colours or different states of polarisation depending on the physical or chemical conditions. A change in colour to another part of the rainbow spectrum may be induced by:

(a) A change in cation concentration such as $H^+$, $Ca^{2+}$, $Mg^{3+}$, or transition metal.

(b) A change in anion concentration such as $Cl^-$ or phosphate.

(c) Covalent modification of the new protein by enzymes causing phospho- or dephosphorylation (including ser/thr, his, and tyr kinases and phosphatases) transglutamination, proteolysis, ADP ribosylation, gly- or glu-cosylation, halogenation, oxidation, methylation and myristilation.

(b) Binding to the rainbow protein of an antigen, an intracellular signal such as cyclic AMP, cyclic GMP, Ip3, Ip4, diacyl glycerol, ATP, ADP, AMP, GTP, any oxy or deoxyribonucleoside or nucleotide, a substrate, a drug, a nucleic acid, a gene regulator protein.

(e) Expression of its nucleic acid inside a live cell, as well as its modification or regulation within the cell by gene expression such as promoters, enhancers or oncogenes.

Single or multiple mutations and deletions may be detected in a piece of DNA (eg PCR product) by linking the "rainbow protein" to one end of the DNA and an energy transfer acceptor or quencher to the other end. Nuclease attack at the mutation will separate the rainbow protein from the acceptor or quencher and thus cause a change in intensity, colour or polarisation of the light emission.

Such alteration, substitution, addition or removal of one or more amino acids may be achieved by chemical means. Alteration of an acid includes alkylation (eg. methylation), phosphorylation and various other covalent modifications of the type outlined herein. Alternatively the nucleic acid coding for the luciferase or photoprotein may be altered by modifying, substituting, inserting or deleting one or more nucleotides such that the resulting protein has gained or lost a site which interacts with the cations, anions, intracellular signal, proteins or nucleic acid to be measured, or undergoes covalent modification. The insertion or deletion of nucleotides is normally produced by site directed mutagenesis or by opening up the gene with a specific restriction enzyme, inserting or deleting a selected nucleotide sequence and then sealing up the gene again or using specific primers with the polymerase chain reaction. The nucleic acid is then transcribed to mRNA and this is then translated to form the rainbow protein either inside a bacterial or eukaryotic cell, or in <u>vitro</u> using, for example, rabbit reticulocyte lysate. The new nucleic acid may contain an RNA polymerase promoter such as T7, SP6, or mammalian promotors such as actin, myosin, myelin proteins, MMT-V, SV40, antibody, G6P dehydrogenase, and can be amplified in <u>vitro</u> using the polymerase chain reaction. The result is that the rainbow protein can be produced either in a live cell such as a cancer cell, or without cells using enzymatic reactions in vitro. The addition of tissue specific promoter or enhancer sequences to the 5' or 3' end of the DNA coding for the native or altered bioluminescent protein will enable it to be used as a reporter gene and to be expressed specifically in a particular cell or tissue, the expression being detectable by the appearance of a change in light intensity, colour or polarisation.

Another way of producing the DNA for a rainbow protein is to separate into two halves the original DNA for the bioluminescent protein. A piece of DNA or gene is then inserted between the two halves by ligating one half to the 5' end and the other to the 3' end. Alternatively the rainbow protein DNA could be generated using the polymerase chain reaction so that the sense primer had one part of the rainbow protein DNA linked at the 5' end and the antisense primer and the other part linked at the 3' end (i.e. antisense). The pieces of DNA or gene of interest, in the middle, could be from two separate genes. For example one could code for an energy transfer protein, the other for a bioluminescent protein. Only when the two are linked together via a peptide (from DNA/RNA) will the rainbow protein be generated and a shift in colour occur. The energy transfer protein could be any fluor bound covalently or non-covalently to the protein, for example the green fluorescent protein from Aequorea, Obelia, Renilla or other cnidarians, or the blue or yellow fluorescent protein from luminous bacteria, or a flavoprotein, or a phycobiliprotein. The whole protein or just the fluorescent domain may be used. The bioluminescent protein could be any luciferase for example bacterial, firefly, glowworm or copepod, or any photoprotein for example aequorin, obelin or a radiolarian such as thalassicollin.

The protein or its DNA or RNA may be incorporated into a live bacterium or eukaryotic cell by using a virus, plasmid, calcium phosphate transfection, electroporation, liposome fusion or membrane pore forming proteins. Once inside, only live cells with the appropriate biochemistry will produce the "rainbow effect". By incorporating the "rainbow protein" gene into an embryo, oocyte, sperm, seed or seedling a transgenic animal or plant may be produced, enabling gene expression, cell regulation, drug action, or cell damage to be located and measured in individual organs using the "rainbow effect". These new organisms may also be used in home aquaria, on aeroplane runways, as safe lights at sea, and as house plants.

The rainbow protein may also be incorporated in a different part of the cell by chemical means or genetically engineering the protein to contain a signal peptide which locates it to the inner or outer surface of the plasma membrane or within a particular intracellular organelle (e.g. peroxisome, mitochondrion, chloroplast, endoplasmic reticulum, golgi, secretory vesicle, nucleus or endosome).

Addition of a signal peptide, either chemically or by genetic engineering, will enable the normal or altered luciferase or photoprotein to be targeted into a specific organelle within the cell, or onto the inner or outer surface of the plasma membrane. For example the sequence MLSRLSLRLLSRYLL at the N terminus will locate the bioluminescent protein in the mitochondria, and KKSALLALMYVCPGKADKE on the N terminus will target the protein to the endoplasmic reticulum, a KDEL sequence at the C terminus retaining it there.

The initial luciferase or photoprotein or its gene may come from any of the known chemistries in bioluminescence (see Figure 1) or from the wide range of uncharacterised luminous organisms from more than 700 genera representing at least 16 phyla. The luciferin may be synthesised chemically and added to the biological reaction or cell to generate light. Alternatively, the gene coding for the enzymes responsible for making the luciferin may be linked to the "rainbow protein" gene so that the artificial operon or fusion gene expresses both rainbow protein and makes luciferin in the live cell from normal cell constituents such as amino acids.

The rainbow protein, or the nucleic acid coding for it, may be used in a range of biological investigations; such as:-

    (a) Detection, location and measurement of microbes (protozoa, bacteria, viruses).
    (b) Detection and location of cancer cells.
    (c) Measurement of enzymes, intracellular signalling and other turnover reactions in cells or fluids.
    (d) DNA and RNA binding assays.
    (e) Immunoassay and other protein binding assays.

The rainbow proteins and their parent nucleic acids also may be used in genetic engineering, in the development of transgenic animals, plants and microbes, and in horticulture.

In this aspect, the reaction or substances of biological interest are made to interact with the rainbow protein or its parent nucleic acid. Such interactions include direct or indirect linking such as non-covalent or covalent binding as well as energy transfer processes.

The invention may be performed in various ways and will be further described with reference to the following examples:

EXAMPLE I

Detection of phosphorylation of a rainbow protein

The peptide leu arg arg ala ser leu gly, known as kemptide, or RTKRSGSVYEPLKT known as malantide was covalently linked to firefly luciferase using disuccinyl suberate at pH8. Addition of 125 ul protein kinase A + cyclic AMP (200 uM) + 125 uM ATP caused phosphorylation of the kemptide, now attached to the luciferase. The resulting shift in colour from yellow-green to red at pH 7.8 or from red to yellow-green at pH 6.5, measured as a ratio in a dual wavelength chemiluminometer, enabled the rate of protein, phosphorylation by the kinase to be assayed, and dephosphorylation induced by phosphatase to be assayed subsequently by the reversal of the ratio.

EXAMPLE 2

Engineering Firefly Luciferase

cDNA coding for firefly luciferase was amplified using the polymerase chain reaction (PCR) using 5' sense primers with a T7 RNA polymerase promoter, and 3' antisense primers as follows: Primer code in brackets

## 5' sense primers

(105)    CACCTAATACGACTCACTATAGGGAGAATGGAAGACGCCAAAAAC

(107)    AGAACTGCCTGCCGCAGATTCTCGCA

(110)    ATGCTGTCCCGGCTGTCCCTGCGGCTGCTGTCCCGGTACCTGCTGAAGACGC

CAAAAAC

(111)    CACCTAATACGACTCACTATAGGGAGAATGCTGTCCCGGCTGTCC


## 3' antisense primers

(100)    TCTCGCTGAATACAGTTAC

(106)    CCCCAAGCTTAGATCTCTCTGATTTTTCTTGCGT

(108)    TGCGAGAATCTGCGGCAGGCAGTTCT


The following firefly luciferase cDNA's were constructed using primers in brackets:

(a) full length (105 + 100)
(b) - 36bp i.e. missing peroxisomal signal peptide (105 + 106)
(c) protein kinase A site (RRXS) in middle of protein (step 1: 105 + 108 and 107 + 100; step 2:2 halves from step 1 + 105 + 100)
(d) mitochondrial signal at N terminus (step 1:110 + 100; step 2: step 1 sample + 111 + 100).

The PCR reaction in 50 $\mu$l contained 10 mM Tris pH 8.3, 0.01% gelatin, 0.4U Taq polymerase, 2 mM MgCl$_2$, 0.2 mM each dATP, dGTP, dTTP, dCTP, 0.5 $\mu$M of each primer, 1 $\mu$l DNA (ca 1-100 ng). The reaction, covered with 50 $\mu$l mineral oil, was incubated in a Perkin-Elmer thermal cycler for 25 cycles: 94$°$C 1 minute, 55$°$C 1 minute, 72$°$C 2 minutes + 5 seconds extension on each cycle, then for 30 minutes at 37$°$C with 1U E.coli DNA polymerase (Klenow fragment).

Successful PCR was confirmed by a band on agarose gel electrophoresis. The cDNA was purified by centricon to remove primers, and precipitated in 70% ethanol, 0.7 mM NH$_4$ acetate after extraction with buffered phenol: CHCl$_3$ : secondary amyl alcohol (25:24:1). The DNA (0.5-1 $\mu$g dissolved in 10mM Tris 0.1 or 1 mM EDTA pH 7.4-7.5) was transcribed in the T7 RNA polymerase in buffer containing 40 mM Tris, pH 7.4-7.5, 6mM MgCl$_2$, 10 mM dithiothreitol, 0.1mg/ml bovine serum albumin, 2 mM spermidine, 0.5 mM each ATP, CTP, UTP, 0.1 mM GTP, 0.5 mM cap m7 G(5') ppp (5') G, 1000 U RNAsin/ml, 800 U T7 RNA polymerase $\pm$ 2 $\mu$C, $^{32}$PUTP for up to 4 hours (1-2 hours optimal), at 37$°$C. The reaction was stopped in the ice cold phenol: CHCl$_3$: secondary amyl alcohol (25:24:1), and the RNA precipitated in 70% ethanol + 0.7M NH$_4$Ac, and stored at -20$°$C.

The RNA was centrifuged, redissolved in 20 $\mu$l 70 mM Tris, 1 mM EDTA pH 7.4-7.5 and 1 $\mu$l incubated with 5-10 $\mu$l rabbit reticulocyte lysate for 1 hour at 30$°$C to synthesize the luciferase. Luciferase, after dilution, 1/100 is assayed for light emission directly in 50 mM tris, 10 mM MgAc$_2$, 0.1 mg/ml bovine serum albumin, 0.1-0.2 mM luciferin, 0.5-5 mM ATP, pH 7.8, or isolated by isoelectric focusing. The mutant (RRXS) luciferase has a pI of ca 7.1 or 6.8, and the normal luciferase pI ca 6.8. The luciferase with mitochondrial signal also separated from the normal luciferase. On addition of the rabbit reticulocyte lysate containing this altered luciferase it was taken up by added mitochondria, as shown by centrifugation and light emission from the mitochondria and luciferase.

Phosphorylation of the RRXS containing luciferase with protein kinase A, cyclic AMP (0.2 mM), ATP (0.1 - 1 mM) pH7, caused the luciferase to change its pI back towards 6.8, and to shift its colour. The RRXS luciferase had a greener light emission than the native luciferase detected using a dual wavelength chemiluminometer with interference filters (maximum transmission ca 545 and 603 mM).

Primers containing kemptide nucleotide sense or antisense sequence (LRRASLG) or malantide RTKRSGSVYEPLKT were also added either to N or C terminus using a one or two step PCR reaction. These also produced luciferase which could be phosphorylated thereby altering its intensity and colour.

EXAMPLE 3

Preparation of engineered aeguorin

cDNA or genomic DNA coding for the $Ca^{2+}$ -activated photoprotein was PCR'd in a similar way to that for firefly luciferase. Using one or two step PCR the protein kinase A recognition peptide kemptide (LRRASLG) or malantide (as Example 2) was added to the N terminus. The mutant aequorin had different kinetic properties enabling protein kinase A to be detected by phosphorylating the altered aequorin (above).

Normal aequorin primers =5' sense TAATACGACTCACTATAGGGGAGAGAATGGTCAAGCTTTACATCAGACTTCGAC, and 3' antisense GAATTCTTAGGGGACAGCTCCACCGTA. For insertion of kemptide at the N terminus the nucleotide sequence equivalent to LRRASLG was attached to the first 15 bases (including ATG) of the 5' end of aequorin. In step 2, the T7 RNA polymerase promoter was added to form the kemptide-aequorin in vitro for in vitro phosphorylation. Genomic aequorin DNA (made by PCR) was at least as active as that made from mRNA by reverse transcriptase PCR.

EXAMPLE 4

Detection of cancer cells in blood

A blood sample (1 ml) is mixed with a suspension of liposomes containing mRNA coding for a rainbow protein catalysing the benzothiazole reaction (c) in Figure 1. This mRNA was produced as follows:-

The gene coding for firefly luciferase was first isolated fron a cDNA library in E. coli using pCDVI plasmid primer + Honio linker containing SP6 RNA polymerase promoter. A nucleotide sequence

$$\text{GCTCGTCTTATTGAAGATAATGAATATACTGCTCGTTTTGGT}$$

representing the phosphorylation site for tyrosine kinase activity of the myc oncogene was inserted at the EcoRI restriction site 30 base pairs downstream from the ATG at the 5' end. The DNA was resealed, recloned and the plasmid insert transcribed by SP6 RNA polymerase in vitro to produce mRNA for the rainbow protein.The original protein produced yellow-green light but the rainbow protein when phosphorylated in the cell produces red light. Thus the presence of cancer cells was detected in blood sample from a leukaemia patient by measuring the ratio of yellow-green (545 nm) to red (603 nm) light in a dual wavelength chemiluminometer.

EXAMPLE 5

Detection of Salmonella

The cDNA for the rainbow protein in Example 4 containing SP6 RNA polymerase promoter was inserted into Salmonella phage. Addition of this phage to Salmonella resulted in expression of the rainbow protein and the generation of red light, enabling as few as 1 bacterium per 20 ml to be detected.

EXAMPLE 6

Detection of HIV RNA

A sample (1 ml) of blood from a patient with AIDS was extracted with 4 M guanidium isothiocyanate and the nucleic acid precipitated with ethanol/NH acetate. An oligonucleotide (10 ul, 1 uM) labelled with a rainbow protein generated from the photoprotein obelin was added to 100 ul redissolved RNA at 50°C and the mixture cooled for 10 minutes at 0°C. The oligonucleotide was specific for a sequence in the HIV coat protein. Binding this to HIV RNA resulted in a shift in the light emission from the rainbow protein from light blue (475 nm) to blue (440 nm). This was detected as a shift in the ratio of light emission at these two wavelengths in a dual photomultiplier chemiluminometer.

EXAMPLE 7

Measurement of testosterone in blood

Testosterone carboxyoxime is reacted with the rainbow protein from the photoprotein obelin to form a testosterone rainbow protein conjugate. 5ul of this containing 1 nmol was incubated with a solution of antibody labelled with fluorescein to testosterone (50ul) pH 7.4 for 30 minutes in the presence or absence of varying concentrations of standard testosterone. The bioluminescent reaction was triggered by addition of $Ca^{2+}$ and the ratio of light at 475 nm to 530 nm measured. Increasing the concentration of standard testosterone increased the ratio at 475/530. This procedure could be carried out without the need to separate bound from free antigen.

EXAMPLE 8

Detection of Listeria

A sample of suspect food is boiled to extract DNA. A sense primer to the specific Listeria gene or domain covalently coupled to obelin cDNA + 5P6 RNA polymerase promotor, and an antisense primer covalently coupled to antisense green fluorescent protein (GFP) cDNA is used to amplify the Listeria gene using the polymerase chain reaction. The result is DNA coding for a new rainbow protein and transcribable by SP6 RNA polymerase.

```
                    Obelin      Listeria      GFP

    1---------1----------1--------1--------1--------------

      promotor             cDNA        gene         cDNA
```

This DNA is transcribed and the mRNA translated using rabbit reticulocyte lysate. Coelenterazine is added to reactivate obelin. The ratio of light at 510/475 nm for rainbow protein versus obelin alone is directly proportional to the amount of Listeria DNA originally present in the food sample. When no Listeria are present the ratio of ratios is 1.

EXAMPLE 9

Measurement of nucleic acid hybridisation by polarisation

The reaction described in Example 6 was carried out but the light emission was detected in a dual photomultiplier chemiluminometer containing two plane polarised filters with the polarisation planes at 90° to each other. The ratio between the two photomultipliers was related to the amount of HIV RNA present.

EXAMPLE 10

Measurement of cyclic AMP or Ip3

Using a two step PCR reaction as described in Examples 2 and 3, the cyclic AMP binding domain from the bacterial CAP protein or the Ip3 binding domain of the endoplasmic reticulum receptor was added to the N or C terminus or into firefly luciferase or aequorin. The altered proteins were made in vitro from the PCR DNA product as described in Examples 2 and 3, and characterised by activity and colour of light emission as cyclic AMP or Ip3. A change in both intensity and colour enabled cAMP or Ip3 to be measured in cell extracts in living cells. using an image intensifier a cAMP or IP3 "cloud" could be visualised in this one cell. Similarly the aequorin or luciferase could be seen within the ER or a mitochondrion if it was first made with an EP. or mitochondrial signal attached to it (±KDEL) at the C terminus.

It will be appreciated that the bioluminescent protein may be synthesised from amino acid sequences or using DNA or RNA synthesis techniques, instead of by modification of a protein produced by an organism.

**Claims**

1.    A bioluminescent protein selected from the group comprising firefly luciferase, aequorin or obelin, modified to include at the N or C terminus or within the protein a recognition site for a selected analyte, said recognition site being selected from the group comprising kemptide, malantide and an RRXS phosphorylation site, and not being present in the unmodified protein, wherein the analyte is capable of interacting with said recognition site, the modified bioluminescent protein being reactive in a bioluminescent reaction to produce light or other radiation, wherein the light or other radiation is produced having a physical property of a first characteristic when the analyte is interacting with the recognition site, and is produced having a second different characteristic of said physical property when the analyte is not interacting with said recognition site.

2. A bioluminescent protein selected from the group comprising firefly luciferase, aequorin and obelin, modified to include at the N or C terminus or within the protein a recognition site responsive to the ambient conditions, said recognition site being selected from the group comprising kemptide, malantide and an RRXS phosphorylation site, and not being present in the unmodified protein, the modified bioluminescent protein being reactive in a bioluminescent reaction to produce light or other radiation, wherein the light or other radiation produced has a physical property of a first characteristic when the recognition site is exposed to a first condition, and has a second different characteristic when said recognition site is exposed to a second condition.

3. A bioluminescent protein according to Claim 1 or 2, wherein said first and second characteristics include at least one of colour, polarization state and intensity.

4. A bioluminescent protein selected from the group comprising firefly luciferase, aequorin and obelin, modified to include at the N or C terminus or within the protein a recognition site responsive to the concentration of a substance of interest, said site being selected from the group comprising kemptide, malantide and an RRXS phosphorylation site, and not being present in the unmodified protein, said modified bioluminescent protein being reactive in a bioluminescent reaction wherein light or other radiation emitted in a bioluminescent reaction involving said protein is produced in a varying physical characteristic depending on the concentration of said substance of interest.

5. A bioluminescent protein according to any of Claims 1 to 4, wherein said bioluminescent protein includes a signal peptide for targeting the protein to a preselected organelle or site within the cell.

6. A bioluminescent protein according to any of Claims 1 to 5, comprising firefly luciferase modified to include kemptide covalently bound to the N or C terminus thereof.

7. A bioluminescent protein according to any of Claims 1 to 5, comprising firefly luciferase modified to include malantide covalently bound to the N or C terminus thereof.

8. A bioluminescent protein according to any of Claims 1 to 5, comprising firefly luciferase modified to include an RRXS phosphorylation site intermediate the N and C terminus.

9. A bioluminescent protein according to any of Claims 1 to 5, comprising aequorin modified to include kemptide covalently bound at the N terminus.

10. A bioluminescent protein according to any of Claims 1 to 5, comprising aequorin modified to include malantide covalently bound at the N terminus.

11. A method of detecting, locating or measuring a substance of interest, the method comprising:-

(a) providing a bioluminescent protein according to any of the preceding claims in a reaction system containing an analyte of interest,
(b) causing said bioluminescent protein to take part in a bioluminescent reaction, and
(c) analyzing the characteristics of light or other radiation produced, and
(d) detecting, locating or measuring said analyte, based on said characteristics.

12. A method of detecting a change in a physical, chemical, biochemical or biological condition, the method comprising:-

(a) providing a bioluminescent protein according to any of Claims 1 to 10 in a reaction system;
(b) causing said bioluminescent protein to take part in a bioluminescent reaction;
(c) observing whether light or other radiation of the first and/or second characteristic is produced, and

(d) detecting said change based on production of light or radiation of said first or second characteristic.

13. A method of detecting, locating or measuring a substance of interest within a cell, or monitoring at least one of the physical, chemical, biochemical or biological conditions within a cell, which method comprises:

(a) introducing into said cell nucleic acid coding for a bioluminescent protein according to any of Claims 1 to 10,
(b) causing said bioluminescent protein to be expressed;
(c) causing said modified bioluminescent protein to take part in a bioluminescent reaction, and
(d) analyzing the characteristics of the light or other radiation produced.

14. Nucleic acid coding for the bioluminescent protein as claimed in any of Claims 1 to 10.

15. Nucleic acid according to Claim 14, including a promoter or enhancer sequence specific to a tissue or substance of interest.

**Patentansprüche**

1. Biolumineszierendes Protein aus der Gruppe Leuchtkäfer-Luciferase, Aequorin oder Obelin, welches derart modifiziert ist, daß es am N- oder C-Ende oder innerhalb des Proteins eine Erkennungsstelle für eine ausgewählte Analysensubstanz aufweist, wobei diese Erkennungsstelle aus der Gruppe Kemptid, Malantid und einer RRXS Phosphorylationsstelle gewählt ist und im unmodifizierten Protein nicht vorhanden ist, wobei die Analysensubstanz mit der Erkennungsstelle wechselwirkt, wobei das modifizierte, biolumineszierende Protein derart reaktiv ist, daß es in einer Biolumineszenzreaktion Licht oder eine andere Strahlung erzeugt, wobei das erzeugte Licht oder die andere erzeugte Strahlung eine physikalische Eigenschaft mit erster Charakteristik aufweist, wenn die Analysensubstanz mit der Erkennungsstelle wechselwirkt, und eine zweite unterschiedliche Charakteristik dieser physikalischen Eigenschaft aufweist, wenn die Analysensubstanz nicht mit der Erkennungsstelle wechselwirkt.

2. Biolumineszierendes Protein aus der Gruppe Leuchtkäfer-Luciferase, Aequorin oder Obelin, welches derart modifiziert ist, daß es am N- oder C-Ende oder innerhalb des Proteins eine Erkennungsstelle aufweist, welche auf Umgebungszustände anspricht, wobei diese Erkennungsstelle aus der Gruppe Kemptid, Malantid und einer RRXS Phosphorylationsstelle gewählt ist und im unmodifizierten Protein nicht vorhanden ist, wobei das modifizierte, biolumineszierende Protein derart reaktiv ist, daß es in einer Biolumineszenzreaktion Licht oder eine andere Strahlung erzeugt, wobei das erzeugte Licht oder die andere erzeugte Strahlung eine physikalische Eigenschaft mit erster Charakteristik aufweist, wenn die Erkennungsstelle einem ersten Zustand ausgesetzt ist, und eine zweite, unterschiedliche Charakteristik dieser physikalischen Eigenschaft aufweist, wenn die Erkennungsstelle einem zweiten Zustand ausgesetzt ist.

3. Biolumineszierendes Protein nach Anspruch 1 oder 2, **dadurch gekennzeichnet, daß** die erste und zweite Charakteristik wenigstens eines Farbe, einen Polarisationszustand oder eine Intensität umfaßt.

4. Biolumineszierendes Protein aus der Gruppe Leuchtkäfer-Luciferase, Aequorin oder Obelin, welches derart modifiziert ist, daß es am N- oder C-Ende oder innerhalb des Proteins eine Erkennungsstelle aufweist, welche auf einen Konzentration einer interessierenden Substanz anspricht, wobei diese Erkennungsstelle aus der Gruppe Kemptid, Malantid und einer RRXS Phosphorylationsstelle gewählt ist und im unmodifizierten Protein nicht vorhanden ist, wobei das modifizierte, biolumineszierende Protein in einer Biolumineszenzreaktion Licht oder eine andere Strahlung erzeugt, wobei das bei der Biolumineszenzreaktion mit dem Protein erzeugte Licht oder die erzeugte Strahlung in Abhängigkeit von der Konzentration der interessierenden Substanz variierende physikalische Eigenschaften aufweist.

5. Biolumineszierendes Protein nach einem der Ansprüche 1 bis 4, **dadurch gekennzeichnet, daß** dieses ein Signalpeptid zum gezielten Hinführen des Proteins an eine vorbestimmte Organelle oder Stelle innerhalb der Zelle aufweist.

6. Biolumineszierendes Protein nach einem der Ansprüche 1 bis 5, **dadurch gekennzeichnet, daß** dieses Leuchtkäfer-Luciferase aufweist und derart modifiziert ist, daß es Kemptid umfaßt, welches kovalent an dessen N- oder C-Ende gebunden ist.

**7.** Biolumineszierendes Protein nach einem der Ansprüche 1 bis 5, **dadurch gekennzeichnet, daß** dieses Leuchtkäfer-Luciferase aufweist und derart modifiziert ist, daß es Malantid umfaßt, welches kovalent an dessen N- oder C-Ende gebunden ist.

**8.** Biolumineszierendes Protein nach einem der Ansprüche 1 bis 5, **dadurch gekennzeichnet, daß** dieses Leuchtkäfer-Luciferase aufweist und derart modifiziert ist, daß es zwischen dem N- und dem C-Ende eine RRXS Phosphorylationsstelle umfaßt.

**9.** Biolumineszierendes Protein nach einem der Ansprüche 1 bis 5, **dadurch gekennzeichnet, daß** dieses Aequorin aufweist und derart modifiziert ist, daß es Kemptid umfaßt, welches kovalent an dessen N- oder C-Ende gebunden ist.

**10.** Biolumineszierendes Protein nach einem der Ansprüche 1 bis 5, **dadurch gekennzeichnet, daß** dieses Aequorin aufweist und derart modifiziert ist, daß es Malantid umfaßt, welches kovalent an dessen N- oder C-Ende gebunden ist.

**11.** Verfahren zum Detektieren, Lokalisieren oder Messen einer interessierenden Substanz mit folgenden Schritten:

(a) Zurverfügungstellen eines biolumineszierenden Proteins gemäß wenigstens einem der vorhergehenden Ansprüche in einem Reaktionssystem, welches eine interessierende Analysensubstanz enthält,
(b) Herbeiführen einer biolumineszierenden Reaktion des biolumineszierenden Proteins,
(c) Analysieren der Charakteristiken von erzeugtem Licht oder anderer Strahlung und
(d) Detektieren, Lokalisieren oder Messen der Analysensubstanz auf der Basis der analysierten Charakteristiken.

**12.** Verfahren zum Detektieren einer Änderung eines physikalischen, chemischen, biochemischen oder biologischen Zustands mit folgenden Schritten:

(a) Zurverfügungstellen eines biolumineszierenden Proteins gemäß wenigstens einem der Ansprüche 1 bis 10 in einem Reaktionssystem,
(b) Herbeiführen einer biolumineszierenden Reaktion des biolumineszierenden Proteins,
(c) Beobachten ob Licht oder eine andere Strahlung einer ersten und/oder zweiten Charakteristik erzeugt wird und
(d) Detektieren der Änderung auf der Basis des erzeugten Lichtes oder anderer Strahlung mit erster oder zweiter Charakteristik.

**13.** Verfahren zum Detektieren, Lokalisieren oder Messen einer interessierenden Substanz innerhalb einer Zelle oder Überwachen wenigstens eines physikalischen, chemischen, biochemischen oder biologischen Zustands innerhalb einer Zelle mit folgenden Schritten:

(a) Einführen eines Nukleinsäurekodes für ein biolumineszierendes Protein gemäß wenigstens einem der Ansprüche 1 bis 10 in die Zelle,
(b) Herbeiführen der Darstellung des biolumineszierenden Proteins,
(c) Herbeiführen einer biolumineszierenden Reaktion des modifizierten biolumineszierenden Proteins,
(d) Analysieren der Charakteristiken von erzeugtem Licht oder anderer Strahlung.

**14.** Nukleinsäurekodierung für ein biolumineszierendes Protein gemäß wenigstens einem der Ansprüche 1 bis 10.

**15.** Nukleinsäurekodierung nach Anspruch 14, **dadurch gekennzeichnet, daß** eine Promoter- oder Enhancer-Sequenz vorgesehen ist, welche für ein Gewebe oder eine interessierende Substanz spezifisch ist.

**Revendications**

**1.** Protéine bioluminescente choisie dans le groupe comprenant la luciférase de luciole, l'aequorine ou l'obéline, modifiée pour comprendre à l'extrémité N- ou C-terminale ou à l'intérieur de la protéine un site de reconnaissance pour un analyte sélectionné, ledit site de reconnaissance étant choisi dans le groupe comprenant le kemptide, le malantide et un site de phosphorylation RRXS, et n'étant pas présent dans la protéine non modifiée, l'analyte étant capable d'interagir avec ledit site de reconnaissance, la protéine bioluminescente modifiée étant réactive dans une réaction bioluminescente pour produire de la lumière ou une autre radiation, où la lumière ou l'autre radiation est produite ayant une propriété physique d'une première caractéristique lorsque l'analyte est en interaction avec le site de reconnaissance, et est produite ayant une seconde caractéristique différente de ladite propriété physique lorsque l'analyte n'est pas en interaction avec ledit site de reconnaissance.

2. Protéine bioluminescente choisie dans le groupe comprenant la luciférase de luciole, l'aequorine et l'obéline, modifiée pour comprendre à l'extrémité N- ou C-terminale ou à l'intérieur de la protéine un site de reconnaissance sensible aux conditions ambiantes, ledit site de reconnaissance étant choisi dans le groupe comprenant le kemptide, le malantide et un site de phosphorylation RRXS, et n'étant pas présent dans la protéine non modifiée, la protéine bioluminescente modifiée étant réactive dans une réaction bioluminescente pour produire de la lumière ou une autre radiation, où la lumière ou l'autre radiation produite a une propriété physique d'une première caractéristique lorsque le site de reconnaissance est exposé à une première condition, et a une seconde caractéristique différente lorsque ledit site de reconnaissance est exposé à une seconde condition.

3. Protéine bioluminescente selon l'une des revendications 1 ou 2, dans laquelle lesdites première et seconde caractéristiques comprennent au moins l'une parmi la couleur, l'état de polarisation et l'intensité.

4. Protéine bioluminescente choisie dans le groupe comprenant la luciférase de luciole, l'aequorine et l'obéline, modifiée pour comprendre à l'extrémité N- ou C-terminale ou à l'intérieur de la protéine un site de reconnaissance sensible à la concentration d'une substance d'intérêt, ledit site étant choisi dans le groupe comprenant le kemptide, le malantide et un site de phosphorylation RRXS, et n'étant pas présent dans la protéine non modifiée, ladite protéine bioluminescente modifiée étant réactive dans une réaction bioluminescente dans laquelle la lumière ou une autre radiation émise dans une réaction bioluminescente mettant en jeu ladite protéine est produite dans une caractéristique physique variable dépendant de la concentration de ladite substance d'intérêt.

5. Protéine bioluminescente selon l'une quelconque des revendications 1 à 4, dans laquelle ladite protéine bioluminescente comprend un peptide signal pour cibler la protéine sur un organite ou site présélectionné à l'intérieur de la cellule.

6. Protéine bioluminescente selon l'une quelconque des revendications 1 à 5, comprenant la luciférase de luciole modifiée pour comprendre le kemptide lié de façon covalente à l'extrémité N- ou C-terminale de celle-ci.

7. Protéine bioluminescente selon l'une quelconque des revendications 1 à 5, comprenant la luciférase de luciole modifiée pour comprendre le malantide lié de façon covalente à l'extrémité N- ou C-terminale de celle-ci.

8. Protéine bioluminescente selon l'une quelconque des revendications 1 à 5, comprenant la luciférase de luciole modifiée pour comprendre un site de phosphorylation RRXS intermédiaire entre l'extrémité N-terminale et l'extrémité C-terminale.

9. Protéine bioluminescente selon l'une quelconque des revendications 1 à 5, comprenant de l'aequorine modifiée pour comprendre le kemptide lié de façon covalente à l'extrémité N-terminale.

10. Protéine bioluminescente selon l'une quelconque des revendications 1 à 5, comprenant de l'aequorine modifiée pour comprendre le malantide lié de façon covalente à l'extrémité N-terminale.

11. Procédé de détection, de localisation ou de mesure d'une substance d'intérêt, le procédé comprenant les opérations consistant :

   (a) disposer une protéine bioluminescente telle que définie à l'une quelconque des revendications précédentes dans un système de réaction contenant un analyte d'intérêt ;
   (b) amener ladite protéine bioluminescente à prendre part à une réaction bioluminescente ; et
   (c) analyser les caractéristiques de la lumière ou autre radiation produite ; et
   (d) détecter, localiser ou mesurer ledit analyte, sur la base desdites caractéristiques.

12. Procédé de détection d'un changement dans un état physique, chimique, biochimique ou biologique, le procédé comprenant les opérations consistant à :

(a) disposer une protéine bioluminescente telle que définie à l'une quelconque des revendications 1 à 10 dans un système réactionnel ;

(b) amener ladite protéine bioluminescente à prendre part à une réaction bioluminescente ;

(c) observer si ou non la lumière ou une autre radiation de la première et/ou seconde caractéristique est produite ; et

(d) détecter ledit changement sur la base de la production de lumière ou de radiation de ladite première ou seconde caractéristique.

13. Procédé de détection, de localisation ou de mesure d'une substance d'intérêt à l'intérieur d'une cellule, ou de surveillance d'au moins l'une des conditions physique, chimique, biochimique ou biologique à l'intérieur d'une cellule, lequel procédé comprend les opérations consistant à :

(a) introduire dans ladite cellule de l'acide nucléique codant pour une protéine bioluminescente telle que définie à l'une quelconque des revendications 1 à 10 ;

(b) amener ladite protéine bioluminescente à être exprimée ;

(c) amener ladite protéine bioluminescente modifiée à prendre part à une réaction bioluminescente ; et

(d) analyser les caractéristiques de la lumière ou autre radiation produite.

14. Acide nucléique codant pour la protéine bioluminescente telle que définie à l'une quelconque des revendications 1 à 10.

15. Acide nucléique selon la revendication 14, comprenant une séquence promoteur ou amplificatrice spécifique d'un tissu ou d'une substance d'intérêt.

# 1. ALDEHYDES

$CH_3(CH_2)_n CHO + FMN-OOH \; n>7$  bacteria

OCHO

Latia
(limpet)

O
CHO
N
H

Diplocardia
(earthworm)

# 2. IMIDAZOLOPYRAZINES

HO
N
N
OH

coelenterates
decapods,
mysids, squid
copepods,
radiolarians
some fish

ostracods

NH
NH—C
NH2

# 3. BENZOTHIAZOLES

O
N        N
OAMP
HO        S        S

coleoptera
(beetles)

# 4. TETRAPYRROLES

COOR

O
N        N        N        N
H        H        H
O        O

dinoflagellates

CHO        COOR

O
HO
N        N        N        N
H        H        H
O        O

euphausiids

Analogue of
— Cys —| |— Ser —
O
$CO_2H\,C\cdot O$
S        CH3        CH2        CH2        CH3
CH3 CH        CH3 CH2        CH2 CH3        CH3 CH2
H
O        N        C        N        C        N        C        N        O
H        H        H        H        H        H

Malacosteidae

# 5. FLAVINS
bacteria, fungi  scaleworms

FIG. 1